# EUROPEAN PATENT SPECIFICATION

(11) **EP 1 971 332 B1**
(45) Date of publication and mention of the grant of the patent: **09.03.2011**
(21) Application number: 06830792.5
(22) Date of filing: 22.12.2006
(51) Int. Cl.: A61K 31/381, A61P 11/06

(54) **USE OF TIOTROPIUM SALTS IN THE TREATMENT OF MODERATE PERSISTENT ASTHMA**
VERWENDUNG VON TIOTROPIUM-SALZEN BEI DER BEHANDLUNG VON MÄSSIGEM PERSISTIERENDEM ASTHMA
UTILISATION DES SELS DE TIOTROPIUM DANS LE TRAITEMENT DE L'ASTHME MODÉRÉ PERSISTANT

(30) Priority: 04.01.2006 EP 06100055
(43) Date of publication of application: 24.09.2008
(73) Proprietor: Boehringer Ingelheim International GmbH, 55216 Ingelheim am Rhein (DE); Boehringer Ingelheim Pharma GmbH & Co. KG, 55216 Ingelheim am Rhein (DE)
(72) Inventor: ENGEL, Michael, 89077 Ulm (DE); HEINRICHS, Stefan, 60389 Frankfurt (DE)
(74) Representative: Hammann, Heinz
(86) International application number: PCT/EP2006/070135
(87) International publication number: WO 2007/077162

(56) References cited:
- US-A1- 2003 119 802
- US-A1- 2005 143 410
- US-A1- 2005 256 149
- BARNES: "Tiotropium Bromide" EXP.OPINION INVEST.DRUGS, vol. 10, no. 4, 2001, - 2001 pages 733-740, XP002366079

## Description

The instant invention relates to the use of tiotropium salts for the manufacture of a medicament for the treatment of patients suffering from moderate persistent asthma.

### Background of the invention

Asthma is one of the most common chronic diseases worldwide. It is a chronic inflammatory disorder of the airways. Asthma causes recurring episodes of wheezing, chest tightness, breathlessness, and coughing. Asthma attacks (or exacerbations) are episodic, but airway inflammation is chronically present. Asthma is known to occur in different severity. Asthma severity can be intermittent, or it can be persistently mild, moderate or severe. Severity varies among individuals, does not necessarily relate to the frequency or persistence of symptoms, and can change in one individual over time. Treatment decisions are made based on severity and vice versa, the severity classification level is based on the medication therapy.

According to worldwide accepted guidelines of GINA (Global initiative for asthma) asthma severity can be classified into so called GINA steps 1 to 4. The severity of asthma determines the treatment to be required (see hereto: GINA - Pocket guide for asthma management and prevention as updated 2005). For many patients, medication must be taken every day to control symptoms, to improve lung function and to prevent attacks. Medications are optionally also required to relieve acute symptoms such as wheezing, chest tightness, and cough.

GINA step 1 asthma is also called intermittent asthma. Symptoms occur usually less than one per week. GINA step 1 asthma does usually not require daily medication.

In mild persistent asthma (GINA step 2) the recommended medication is treatment with low-dose inhaled corticosteroids. For moderate persistent asthma (GINA step 3) administration of low to medium dose corticosteroids in combination with long-acting inhaled beta-2-agonists is recommended.

Finally, severe persistent asthma (GINA step 4) is usually treated with high-dose inhaled corticosteroids in combination with long acting inhaled beta-2-agonists plus one or more of the following if needed: sustained release theophylline, leukotriene modifier, long-acting oral beta-2-agonist, and oral corticosteroids.

It is the object of the invention in hand to provide for an alternative treatment of patients suffering from moderate persistent asthma (GINA step 3). It is another object of the invention to provide for suitable pharmaceutical compositions for the treatment of these patients:

### Description of the invention

The instant invention relates to the use of tiotropium salts **1** for the manufacture of a medicament for the treatment of patients suffering from moderate persistent asthma in the severity GINA step 3

The compound tiotropium bromide is known from European Patent Application EP 418 716 A1 and has the chemical structure: 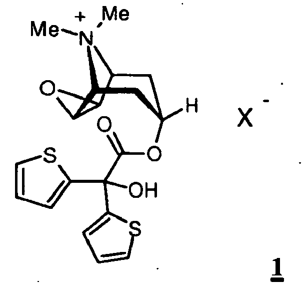 wherein X denotes bromide. Within the scope of the present invention the term tiotropium should be taken as being a reference to the free cation **1**'.

By the tiotropium salts **1** which may be used within the scope of the present invention are meant the compounds which contain, in addition to tiotropium **1**' as counter-ion an anion X with a single negative charge, preferably an anion which is selected from among chloride, bromide, iodide, sulphate, phosphate, methanesulphonate, nitrate, maleate, acetate, citrate, fumarate, tartrate, oxalate, succinate, benzoate and p-toluenesulphonate contain, while the chloride, bromide, iodide, sulphate, methanesulphonate or p-toluenesulphonate are preferred as counter- ions. Of all the salts the chloride, bromide, iodide and methanesulphonate are particularly preferred. Tiotropium bromide is of outstanding importance according to the invention, preferably in form of the crystalline tiotropium bromide monohydrate which is disclosed in WO 02/30928. In another preferred embodiment anhydrous tiotropium bromide as disclosed in WO 03/000265 or in WO 05/042527 is used within the scope of the invention. From these two anhydrous forms the one disclosed in WO 05/042527 is of particular interest.

Within the scope of the invention the term moderate persistent asthma is to be understood as asthma with the severity GINA step 3. Asthma in the severity GINA step 3 is characterised by daily symptoms over a prolonged time or nocturnal asthma more than once a week. A patient with a pre-treatment baseline PEF of more than 60 percent but less than 80 percent of predicted or personal best and PEF variability of 20 to 30 percent. If the patient's asthma is not controlled on a low dose of inhaled glucocorticosteroids (Step 2), then the asthma should also be considered moderate persistent.

PEF (peak expiratory flow) values can be measured with peak flow meters known in the art. Spirometers known in the art are used to measure the so called forced expiratory volume in **1** second (FEV_{L}). The methods for determination of PEF and FEV₁ are well established in the art.

The invention relates to the use of tiotropium salts **1** for the manufacture of a medicament for the treatment of patients suffering from asthma in the severity GINA step 3. The invention also relates to the use of tiotropium salts **1** for the manufacture of a medicament for the treatment of asthma in patients suffering from daily symptoms. The invention also relates to the use of tiotropium salts **1** for the manufacture of a medicament for the treatment of asthma in patients suffering from exacerbations. The invention also relates to the use of tiotropium salts **1** for the manufacture of a medicament for the treatment of asthma in patients suffering from nocturnal asthma symptoms. The invention also relates to the use of tiotropium salts **1** for the manufacture of a medicament for the treatment of asthma in patients with limited physical activity. The invention also relates to the use of tiotropium salts **1** for the manufacture of a medicament for the treatment of asthma in patients with PEF -values 60- 80%. The invention also relates to the use of tiotropium salts **1** for the manufacture of a medicament for the treatment of asthma in patients with FEV₁-values ≤ 80%. The invention also relates to the use of tiotropium salts **1** for the manufacture of a medicament for the treatment of asthma in patients with a PEF variability of 20 to 30%.

Asthmatics with homozygosity for Arg/Arg genotype at codon 16 of the adrenoreceptor beta2 (ADRB2) gene on chromosome 5 do particulary benefit from treatment with tiotropium because the betamimetic is not as effective as in patients with a 'wild-type' (e.g. Gly/Gly) genotype. Therefore, the invention also relates to a method for the treatment of moderate persistent asthma (GINA step 3) in patients with homozygosity for Arg/Arg genotype at codon 16 of the adrenoreceptor beta2 (ADRB2) gene on chromosome 5. In another preferred embodiment the invention relates to a method for the treatment of moderate persistent asthma (GINA step 3) in patients bearing the Arg/Arg SNP (Single Nucleotide Polymorphism). In another preferred embodiment the invention relates to a method for the treatment of moderate persistant asthma (GINA step 3) in patients bearing the Arg/Arg SNP (Single Nucleotide Polymorphism) as a second controller therapy.

In another preferred embodiment the invention relates to a method for the treatment of moderate persistent asthma in GINA step 3 patients with the Arg/Arg genotype.

The term "homozygosity for Arg/Arg genotype at codon 16 of the adrenoreceptor beta2 (ADRB2) gene on chromo some 5" is to be understood as the description of a patient population, which is affected by a single nucleotide mutation in an extent of about 15-20% of the general population. A number of studies have reported associations of ADRB2 polymorphisms with asthma. Recent studies revealed for patients who were homozygous (i.e. alleles at a genetic locus are identical) for arginine at the 16^{th} amino acid position a detrimental response to regular β2-agonist use.

GINA step 3 patients with the Arg/Arg genotype are patients who are still instructed to use low-to-medium inhaled glucocorticosteroids plus a long acting inhaled beta-2-agonist - according to the existing guidelines.

Patients bearing the Arg/Arg SNP (Single Nucleotide Polymorphism) are patients assumed to have an inadequate treatment response to a second controller therapy such as long-acting beta-2-agonists. There is evidence that tiotropium bromide as a long acting anticholineraic may be a treatment option in patients with moderate persistent asthma and will be administered as an alternative controller medication without having severe adverse side effects.

The term "therapeutically effective amount" shall mean that amount of a drug or pharmaceutical agent that will elicit the biological or medical response of a tissue, system, animal or human that is being sought by a researcher or clinician.

For moderate and persistent asthma medical treatment with corticosteroids is recommended. However, patients suffering from moderate persistent asthma do often show daily symptoms over a prolonged time despite of a treatment with inhaled corticosteroids. In another embodiment the invention, therefore, relates to the use of tiotropium salts **1** for the manufacture of a medicament for the treatment of moderate persistent asthma in patients showing persistent symptoms despite of treatment with inhaled corticosteroids.

For moderate and persistent asthma medical treatment with beta-2-agonists, in particular with inhaled long-acting beta-2-agonists is also recommended. However, patients suffering from moderate persistent asthma do often show daily symptoms over a prolonged time despite of a treatment with inhaled beta-2-agonists. In another embodiment the invention, therefore, relates to the use of tiotropium salts **1** for the manufacture of a medicament for the treatment of moderate persistent asthma in patients showing persistent symptoms despite of treatment with inhaled beta-2-agonists.

For moderate and persistent asthma medical treatment with corticosteroids in combination with long-acting beta-2-agonists is recommended as primary controller therapy. However, patients suffering from moderate persistent asthma do often show daily asthma symptoms despite of a treatment with inhaled corticosteroids in combination with long-acting beta-2-agonists. In another embodiment the invention, therefore, relates to the use of tiotropium salts **1** for the manufacture of a medicament for the treatment of moderate persistent asthma in patients showing daily symptoms despite of combined treatment with inhaled corticosteroids and long-acting beta- 2-agonists.

In a yet another preferred embodiment, the invention relates to the use of tiotropium salts **1** for the manufacture of a medicament for the maintenance treatment of moderate persistent asthma and the prevention of broncho-obstructive symptoms in patients who are not adequately controlled by maintenance controller treatment with inhaled corticosteroids and long-acting beta-2-agonists.

In a yet another preferred embodiment, the invention relates to the use of tiotropium salts **1** for the manufacture of a medicament for the maintenance treatment of asthma in the severity GINA step 3 and the prevention of broncho-obstructive symptoms in patients who are not adequately controlled by maintenance controller treatment with inhaled corticosteroids and long-acting beta-2-agonists.

In a yet another preferred embodiment, the invention relates to the use of tiotropium salts **1** for the manufacture of a medicament for the second-line maintenance controller therapy for the treatment of moderate persistent asthma.

In a yet another preferred embodiment, the invention relates to the use of tiotropium salts **1** for the manufacture of a medicament for the maintenance treatment of moderate persistent asthma and the prevention of broncho-obstructive symptoms in patients who receive already maintenance controller treatment with inhaled corticosteroids.

In a yet another preferred embodiment, the invention relates to the use of tiotropium salts **1** for the manufacture of a medicament for the maintenance treatment of asthma in the severity GINA step 3 and the prevention of broncho-obstructive symptoms in patients who receive already maintenance controller treatment with inhaled corticosteroids.

In a yet another preferred embodiment, the invention relates to the use of tiotropium salts **1** for the manufacture of a medicament for the maintenance treatment of moderate persistent asthma and the prevention of broncho-obstructive symptoms in patients who receive already maintenance controller treatment with inhaled corticosteroids, wherein the inhaled corticosteroid is selected from among prednisolone, prednisone, butixocortpropionate, RPR-106541, flunisolide, beclomethasone, triamcinolone, budesonide, fluticasone, mometasone, ciclesonide, rofleponide, ST-126, dexamethasone, (S)-fluoromethyl 6α,9a-difluoro-7α-[(2-furanylcarbonyl)oxy] -11β-hydroxy-16α-methyl-3-oxo-androsta-1,4-diene-17β-carbothionate, (S)-(2-oxo-tetrahydro-furan-3S-yl)6α,9α-difluoro-11β-hydroxy-16α-methyl-3-oxo- 17α-propionyloxy-androsta-1,4-diene- 17β-carbothionate and etiprednol-dichloroacetate (BNP-166,), optionally in the form of the racemates, enantiomers or diastereomers thereof and optionally in the form of the salts and derivatives thereof, the solvates and/or hydrates thereof.

In a yet another preferred embodiment, the invention relates to the use of tiotropium salts **1** for the manufacture of a medicament for the maintenance treatment of severe persistent asthma and the prevention of broncho-obstructive symptoms in patients who receive already maintenance controller treatment with inhaled corticosteroids, wherein the inhaled corticosteroid is selected from among flunisolide, beclomethasone, triamcinolone, budesonide, fluticasone, mometasone, ciclesonide, rofleponide, ST-126, dexamethasone, (S)-fluoromethyl 6α,9α-difluoro-17α-[(2-furanylcarbonyl)oxy]-11β-hydroxy-16α-methyl-3-oxo-androsta-1,4-diene-17β-carbothionate, (S)-(2-oxo-tetrahydro-furan-3S-yl)6α,9α-difluoro-11β-hydroxy-16α-methyl-3-oxo-17α-propionyloxy-androsta-1,4-diene-17-β-carbothionate and etiprednol-dichloroacetate, optionally in the form of the racemates, enantiomers or diastereomers thereof and optionally in the form of the salts and derivatives thereof, the solvates and/or hydrates thereof

In a yet another preferred embodiment, the invention relates to the use of tiotropium salts **1** for the manufacture of a medicament for the maintenance treatment of moderate persistent asthma and the prevention of broncho-obstructive symptoms in patients who receive already maintenance controller treatment with inhaled corticosteroids, wherein the inhaled corticosteroid is selected from among budesonide, fluticasone, mometasone, ciclesonide, (S)-fluoromethyl 6α,9α-difluoro- l7α-[(2-furanylcarbonyl)oxy]-11β-hydroxy-16α-methyl-3-oxo-androsta-1,4-diene-17β-carbothionate and etiprednol-dichloroacetate, optionally in the form of the racemates, enantiomers or diastereomers thereof and optionally in the form of the salts and derivatives thereof, the solvates and/or hydrates thereof.

Any reference to steroids includes a reference to any salts or derivatives, hydrates or solvates thereof which may exist. Examples of possible salts and derivatives of the steroids may be: alkali metal salts, such as for example sodium or potassium salts, sulphobenzoates, phosphates, isonicotinates, acetates, propionates, dihydrogen phosphates, palmitates, pivalates or furoates.

In a yet another preferred embodiment, the invention relates to the use of tiotropium salts **1** for the manufacture of a medicament for the maintenance treatment of moderate persistent asthma and the prevention of broncho-obstructive symptoms in patients bearing the Arg/Arg genotype at codon 16 (chromosome 5)who receive already maintenance controller treatment with inhaled corticosteroids and long-acting beta-2-agonists, wherein the long-acting beta-2-agonist is selected from among albuterol, bambuterol, bitolterol, broxaterol, carbuterol, clenbuterol, fenoterol, formoterol, hexoprenaline, ibuterol, isoetharine, isoprenaline, levosalbutamol, mabuterol, meluadrine, metaproterenol, orciprenaline, pirbuterol, procaterol, reproterol, TD 3327, ritodrine, salmeterol, salmefamol, soterenot, sulphonterol, tiaramide, terbutaline, tolubuterol, CHF-4226 (= TA 2005 or carmoterol; HOKU-81, KUL-1248, 3-(4-{6-[2-Hydroxy-2-(4-hydroxy-3-hydroxymethyl-phenyl)-ethylamino]-hexyloxy}-butyl)-benzenesulfoneamide, 5-[2-(5,6-Diethyl- indan-2-ylamino-1-hydroxy-ethyl]-8-hydroxy-1H-quinolin-2-one, 4-hydroxy-7-[2-{[2-{[3-(2-phenylethoxy)propyl]sulphonyl}ethyl]-amino}ethyl]-2(3H)benzothiazolone, 1-(2-fluoro-4-hydroxyphenyl)-2-[4-(1-benzimidazolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy3-oxo-4H-1,4-benzoxazin-8-yl]-2-[3-(4-methoxyphertyl)-2-methyl-2-propylamino]ethanol, 1-[2H-5-hydroxy-3-oxo-4H-1,4-benzoxazin-8-yl]-2-(3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-one, 1-(4-amino-3-chloro-5-trifluormethylphenyl)-2-tert-butylamino)ethanol, 1-(4-ethoxycarbonylamino-3-cyano-5-fluorophenyl)-2-(tert-butylamino)ethanol, and N-[2-Hydroxy-5-(1-hydroxy-2-{2-[4-(2-hydroxy-2-phenyl-ethylamino)-phenyl]-ethylamino}-ethyl)-phenyl]-formamide, optionally in the form of the racemates, the enantiomers, the diastereomers and optionally the pharmacologically acceptable acid addition salts and the hydrates thereof.

In a yet another preferred embodiment, the invention relates to the use of tiotropium salts **1** for the manufacture of a medicament for the maintenance treatment of severe persistent asthma and the prevention of broncho-obstructive symptoms in patients bearing the Arg/Arg genotype at codon 16 (chromosome 5)who receive already maintenance controller treatment with inhaled corticosteroids and long-acting beta-2-agonists, wherein the long-acting beta-2-agonist is selected from among bambuterol, bitolterol, carbuterol, clenbuterol, fenoterol, formoterol, hexoprenaline, ibuterol, purbuterol, procaterol, reproterol, TD 3327, salmeterol, sulphonterol, terbutaline, tolubuterol, CHF-4226 (= TA 2005 or carmoterol;), 3-(4-{6-[2-Hydroxy-2-(4-hydroxy-3-hydroxymethyl-phenyl)-ethylamino]-hexyloxy}-butyl)-benzenesulfoneamide, 5-[2-(5,6-Diethyl-indan-2-ylamino-1-hydroxy-ethyl]-8-hydroxy-1*H*-quinolin-2-one, 4-hydroxy-7-[2-{[2-{[3-(2-phenylethoxy)propyl]sulphonyl}hyl]-amino}ethyl]-2(3Hpbenzothiazolone, 1-(2-fluoro-4-hydroxyphenyl)-2-[4-(1-benzimidazolyl)-2-methyl-2-butylamino]ethanol, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, 5-hydroxy-8-(1-hydroxy-2-isopropylaminobutyl)-2H-1,4-benzoxazin-3-(4H)-one, 1-(4-amino-3-chloro-5-trifluormethylphenyl)-2-tert-butylamino)ethanol, 1-(4-ethoxycarbonylamino-3-cyano-5-fluorophenyl)-2-(tert.;butylamino)ethanol, and N-[2-Hydroxy-5-(1-hydroxy-2-{2-[4-(2-hydroxy-2-phenyl-ethylamino)-phenyl]-ethylamino}-ethyl)-phenyl]-formamide, optionally in the form of the racemates, the enantiomers, the diastereomers and optionally the pharmacologically acceptable acid addition salts and the hydrates thereof.

In a yet another preferred embodiment, the invention relates to the use of tiotropium salts **1** for the manufacture of a medicament for the maintenance treatment of moderate persistent asthma and the prevention of broncho-obstructive symptoms in patients bearing the Arg/Arg genotype at codon 16 (chromosome 5) who receive already maintenance controller treatment with inhaled corticosteroids and long-acting beta-2-agonists, wherein the long-acting beta-2-agonist is selected from among fenoterol, formoterol, salmeterol, CHF-4226 (= TA 2005 or carmoterol:), 3-(4-{6-[2-Hydroxy-2-(4-hydroxy-3-hydroxymethyl-phenyl)-ethylamino]-hexyloxy}-butyl)-benzenesulfoneamide, 5-[2-(5,6-Diethyl-indan-2-ylamino)-1-hydioxy-ethyl]-8-hydroxy-1*H*-quinolin-2-one, 1-[3-(4-methoxybenzyl-amino)-4-hydroxyphenyl]-2-[4-(1-benzimidazolyl)-2-methyl-2-butylamino]ethanol, 1-[2H-5-hydroxy-3-oxo-4H-1,4-benzoxazin-8-yl]-2-[3-(4-N,N-dimethylaminophenyl)-2-methyl-2-propylamino]ethanol, 1-[2H-5-hydroxy-3-oxo-4H-1,4-benzoxazin-8-yl]-2-[3-(4-methoxyphenyl)-2-methyl-2-propylamino]ethanol, 1-[2H-5-hydroxy-3-oxo-4H-1,4-benzoxazin-8-yl]-2-[3-(4-n-butyloxyphenyl)-2-methyl-2-propylamino]ethanol, 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol, and N-[2-Hydroxy-5-(1-hydroxy-2-{2-[4-(2-hydroxy-2-phenyl-ethylamino)-phenyl]-ethylamino}-ethyl)-phenyl]-formamide, optionally in the form of the racemates, the enantiomers, the diastereomers and optionally the pharmacologically acceptable acid addition salts and the hydrates thereof. Of the betamimetics mentioned above the compounds formoterol, salmeterol, CHF-4226 (= TA 2005 or carmoterol;), 3-(4-{6-[2-Hydroxy-2-(4-hydroxy-3-hydroxymethy1-phenyl)-ethylamino]-hexyloxy}-butyl)-benzenesulfoneamide, 5-[2-(5,6-Diethyl- indan-2-ylamino)-1-hydroxy-ethyl]-8-hydroxy-1*H*-quinolin-2-one are, and N-[2-Hydroxy-5-(1-hydroxy-2-{2-[4-(2-hydroxy-2-phenyl-ethylamino)-phenyl]-ethylamino}-ethyl)-phenyl]-formamide, particularly preferred, optionally in the form of the racemates, the enantiomers, the diastereomers and optionally the pharmacologically acceptable acid addition salts thereof, and the hydrates thereof.

Examples of pharmacologically acceptable acid addition salts of the beta-2-agonist according to the invention are the pharmaceutically acceptable salts which are selected from among the salts of hydrochloric acid, hydrobromic acid, sulphuric acid, phosphoric acid, methanesulphonic acid, acetic acid, fumaric acid, succinic acid, lactic acid, citric acid, tartaric acid, 1-hydroxy-2-naphthalenecarboxylic acid, 4-phenylcinnamic acid, 5-(2.4-difluorophenyl) salicylic acid or maleic acid. If desired, mixtures of the abovementioned acids may also be used to prepare the salts.

According to the invention, the salts of the beta-2-agonist selected from among the hydrochloride, hydrobromide, sulphate, phosphate, fumarate, methanesulphonate, 4-phenylcinnamate, 5-(2.4-difluorophenyl)salicylate, maleate and xinafoate are preferred. Particularly preferred are the salts of in the case of salmeterol selected from among the hydrochloride, sulphate, 4-phenylcinnainate, 5-(2.4-difluorophenyl)salicylate and xinafoate, of which the 4-phenylcinnamate, 5-(2.4-difluorophenyl)salicylate and especially xinafoate are particularly important. Particularly preferred are the salts of in the case of formoterol selected from the hydrochloride, sulphate, hemifumarate and fumarate, of which the hydrochloride, hemifumarate and fumarate are particularly preferred. Of exceptional importance according to the invention is formoterol fumarate dihydrate or formoterol hemifumarate hydrate.

Any reference to the term beta-2-agonist also includes a reference to the relevant enantiomers or mixtures thereof.

In a yet another embodiment the invention, relates to the use of tiotropium salts **1** for the manufacture of a medicament for the treatment of moderate persistent asthma wherein the patient are children, preferably children younger than 14, more preferably younger than 10, even more preferably younger than 8, preferably younger than 6 years of age. In a particular preferred embodiment the children are younger than 5 years of age.

In another aspect the present invention relates to the aforementioned use of tiotropium salts **1** wherein per each individual dose preferably 1 - 20 µg, more preferably 2 - 15 µg of tiotropium 1' are administered. In another aspect the present invention relates to the aforementioned use of tiotropium salts **1** wherein per each individual dose 5 - 10 µg of tiotropium 1' are administered.

In another aspect the present invention relates to the aforementioned use wherein the tiotropium salts **1** are administered once, or twice, preferably once per day. In another aspect the present invention relates to the aforementioned use wherein the tiotropium salts **1** are administered in the morning or in the evening.

Use of tiotropium salts **1** according to the invention includes the use of the solvates and hydrates thus formed, preferably the hydrates, most preferably the monohydrates.

Based on the amounts of the active substance tiotropium 1' administered per single dose as specified hereinbefore the skilled artisan may easily calculate the corresponding amount of for instance tiotropium bromide and/or tiotropium bromide monohydrate.

The tiotropium salts **1** are preferably administered according to the invention by inhalation For this purpose, the tiotropium salts **1** have to be prepared in inhalable formes. Inhalable preparations include inhalable powders, propellant-containing metering aerosols or propellant-free inhalable solutions. Inhalable powders According to the invention containing the tiotropium salts **1** optionally mixed with physiologically acceptable excipients. Within the scope of the present invention, the term propellant-free inhalable solutions also includes concentrates or sterile inhalable solutions ready for use. The formulations which may be used within the scope of the present invention are described in more detail in the next part of the specification.

Inhalable powders which contain 0.01 to 2 % tiotropium are preferred according to the invention. Particularly preferred inhalable powders for use within the invention contain tiotropium in an amount from about 0.03 to 1%, preferably 0.05 to 0.6 %, particularly preferably 0.06 to 0.3 %. Of particular importance according to the invention, finally, are inhalable powders which contain about 0.08 to 0.22 % tiotropium.

The amounts of tiotropium specified above are based on the amount of tiotropium cation contained.

The excipients that are used for the purposes of the present invention are prepared by suitable grinding and/or screening using current methods known in the art. The excipients used according to the invention may also be mixtures of excipients which are obtained by mixing excipient fractions of different mean particle sizes.

Examples of physiologically acceptable excipients which may be used to prepare the inhalable powders for use in the inhalettes according to the invention include monosaccharides (e.g. glucose, fructose or arabinose), disaccharides (e.g. lactose, saccharose, maltose, trehalose), oligo- and polysaccharides (e.g. dextrans, dextrins, maltodextrin, starch, cellulose), polyalcohols (e.g. sorbitol, mannitol, xylitol), cyclodextrins (e.g. α-cyclodextrin, β-cyclodextrin, χ-cyclodextrin, methyl-β-cyclodextrin, hydroxypropyl-β-cyclodextrin), amino acids (e.g. arginine hydrochloride) or salts (e.g. sodium chloride, calcium carbonate), or mixtures thereof. Preferably, mono- or disaccharides are used, while the use of lactose or glucose is preferred, particularly, but not exclusively, in the form of their hydrates. For the purposes of the invention, lactose is the particularly preferred excipient.

Within the scope of the inhalable powders according to the invention the excipients have a maximum average particle size of up to 250µm, preferably between 10 and 150µm, most preferably between 15 and 80µm. It may sometimes seem appropriate to add finer excipient fractions with an average particle size of 1 to 9µm to the excipients mentioned above. These finer excipients are also selected from the group of possible excipients listed hereinbefore. The average particle size may be determined using methods known in the art (cf. for example WO 02/30389, paragraphs A and C). Finally, in order to prepare the inhalable powders according to the invention, micronised crystalline tiotropium bromide anhydrate, which is preferably characterised by an average particle size of 0.5 to 10µm particularly preferably from 1 to 5µm, is added to the excipient mixture (cf. for example WO 02/30389, paragraph B). Processes for grinding and micronising active substances are known from the prior art.

If no specifically prepared excipient mixture is used as the excipient, it is particularly preferable to use excipients which have a mean particle size of 10 - 50 µm and a 10 % fine content of 0.5 to 6 µm.

By average particle size is meant here the 50 % value of the volume distribution measured with a laser diffractometer using the dry dispersion method. The average particle size may be determined using methods known in the art (cf. for example WO 02/30389, paragraphs A and C). Analogously, the 10% fine content in this instance refers to the 10% value of the volume distribution measured using a laser diffractometer. In other words, for the purposes of the present invention, the 10% fine content denotes the particle size below which 10% of the quantity of particles is found (based on the volume distribution).

The percentages given within the scope of the present invention are always percent by weight, unless specifically stated to the contrary.

In particularly preferred inhalable powders the excipient is characterised by a mean particle size of 12 to 35 µm, particularly preferably from 13 to 30 µm.

Also particularly preferred are those inhalable powders wherein the 10 % fine content is about 1 to 4 µm, preferably about 1.5 to 3 µm.

The inhalable powders according to the invention are characterised, in accordance with the problem on which the invention is based, by a high degree of homogeneity in the sense of the accuracy of single doses. This is in the region of < 8 %, preferably < 6 %, most preferably < 4 %.

After the starting materials have been weighed out the inhalable powders are prepared from the excipient and the active substance using methods known in the art. Reference may be made to the disclosure of WO 02/30390, for example. The inhalable powders according to the invention may accordingly be obtained by the method described below, for example. In the preparation methods described hereinafter the components are used in the proportions by weight described in the above-mentioned compositions of the inhalable powders.

First, the excipient and the active substance are placed in a suitable mixing container. The active substance used has an average particle size of 0.5 to 10 µm, preferably 1 to 6 µm, most preferably 2 to 5 µm. The excipient and the active substance are preferably added using a sieve or a granulating sieve with a mesh size of 0.1 to 2 mm, preferably 0.3 to 1 mm, most preferably 0.3 to 0.6 mm. Preferably, the excipient is put in first and then the active substance is added to the mixing container. During this mixing process the two components are preferably added in batches. It is particularly preferred to sieve in the two components in alternate layers. The mixing of the excipient with the active substance may take place while the two components are still being added. Preferably, however, mixing is only done once the two components have been sieved in layer by layer.

The present invention also relates to the use of the inhalable powders according to the invention for preparing a pharmaceutical composition for the treatment of severe persistent asthma as specified hereinbefore.

The inhalable powders according to the invention may for example be administered using inhalers which meter a single dose from a reservoir by means of a measuring chamber (e.g. according to US 4570630A) or by other means (e.g. according to DE 36 25 685 A). Preferably, however, the inhalable powders according to the invention are packed into capsules (to make so-called inhalettes), which are used in inhalers such as those described in WO 94/28958, for example.

Most preferably, the capsules containing the inhalable powder according to the invention are administered using an inhaler as shown for instance in Figure 1 of WO 03/084502 A1, which is herby incorporated by reference. This inhaler is characterized by a housing I containing two windows 2, a deck 3 in which there are air inlet ports and which is provided with a screen 5 secured via a screen housing 4, an inhalation chamber 6 connected to the deck 3 on which there is a push button 9 provided with two sharpened pins 7 and movable counter to a spring 8, and a mouthpiece 12 which is connected to the housing 1, the deck 3 and a cover 11 via a spindle 10 to enable it to be flipped open or shut and airholes 13 for adjusting the flow resistance.

For administering the inhalable powders containing the crystalline tiotropium bromide forms according to the invention using powder-filled capsules it is particularly preferred to use capsules the material of which is selected from among the synthetic plastics, most preferably selected from among polyethylene, polycarbonate, polyester, polypropylene and polyethylene terephthalate. Particularly preferred synthetic plastic materials are polyethylene, polycarbonate or polyethylene terephthalate. If polyethylene is used as one of the capsule materials which is particularly preferred according to the invention, it is preferable to use polyethylene with a density of between 900 and 1000 kg/m³ preferably 940 - 980 kg/m³, more preferably about 960 - 970 kg/m³ (high density polyethylene).

The synthetic plastics according to the invention may be processed in various ways using manufacturing methods known in the art. Injection moulding of the plastics is preferred according to the invention. Injection moulding without the use of mould release agents is particularly preferred. This method of production is well defined and is characterised by being particularly reproducible.

In another aspect the present invention relates to the abovementioned capsules, which contain the above mentioned inhalable powder according to the invention. These capsules may contain about 1 to 20 mg, preferably about 3 to 15 mg, most preferably about 4 to 12 mg of inhalable powder. Preferred formulations according to the invention contain 4 to 6 mg of inhalable powder. Of equivalent importance according to the invention are capsules for inhalation which contain the formulations according to the invention in an amount of from 8 to 12 mg.

The present invention also relates to the use of the abovementioned capsules characterized by a content of inhalable powder according to the invention, for preparing a pharmaceutical composition for treating of moderate persistent asthma as specified hereinbefore.

Filled capsules which contain the inhalable powders according to the invention are produced by methods known in the art, by filling the empty capsules with the inhalable powders according to the invention.

### Examples of inhalable powders

The following Examples serve to illustrate the present invention in more detail without restricting the scope of the invention to the exemplifying embodiments that follow.

The mentioned examples indicate the amount of active ingredient in a powder mixture of 5.5 mg. The person of ordinary skill in the art is able to prepare lager amounts of powder based on the concentration given in the formulations exemplified below.

Besides the active ingredient the mixture contains only the indicated excipient. The mentioned examples can be filled into capsules for inhalation with appropriate inhalers. In the alternative the mentioned examples can be used with multiple dose dry powder inhalers (MDPIs). These MDPIs contain the powder in form of pre-metered doses or not pre-metered, reservoirs. Appropriate devices are known in the art.

### Formulation Example 1:

| | |
|---|---|
| tiotropium bromide monohydrate: | 0.0225 mg |
| lactose monohydrate: | ad 5.5 mg |

### Formulation Example 2:

| | |
|---|---|
| tiotropium bromide: | 0.0226 mg |
| lactose monohydrate: | ad 5.5 mg |

### Formulation Example 3:

| | |
|---|---|
| tiotropium bromide anhydrate: | 0.0225 mg |
| lactose monohydrate: | ad 5.5 mg |

### Formulation Example 4:

| | |
|---|---|
| tiotropium bromide anhydrate: | 0.0111 mg |
| lactose monohydrate: | ad 5.5 mg |

### Formulation Example 5:

| | |
|---|---|
| tiotropium bromide anhydrate: | 0.0226 mg |
| lactose monohydrate:* | ad 5.5 mg |

| | |
|---|---|
| *) the lactose contains 5% specifically added fine content of micronised lactose monohydrate with a mean particle size of about 4µm. | |

### Formulation Example 6:

| | |
|---|---|
| tiotropium bromide monohydrate: | 0.0225 mg |
| lactose monohydrate:* | ad 5.5 mg |

| | |
|---|---|
| *) the lactose contains 5% specifically added fine content of micronised lactose monohydrate with a mean particle size of about 4µm. | |

### Formulation Example 7:

| | |
|---|---|
| tiotropium bromide anhydrate: | 0.0112 mg |
| lactose monohydrate:* | ad 5.5 mg |

| | |
|---|---|
| *) the lactose contains 5% specifically added fine content of micronised lactose monohydrate with a mean particle size of about 4µm. | |

### Propellant-containing aerosol suspensions

The tiotropium salt may optionally also be administered in the form of propellant-containing inhalable aerosols. Aerosol suspensions are particularly suitable for this.

The present invention therefore also relates to suspensions of the crystalline tiotropium bromide forms according to the invention in the propellent gases HFA 227 and/or HFA 134a, optionally combined with one or more other propellent gases, preferably selected from the group consisting of propane, butane, pentane, dimethylether, CHC1F₂ CH₂F₂, CF₃CH₃, isobutane, isopentane and neopentane.

According to the invention those suspensions which contain as propellent gas only HFA 227, a mixture of HFA 227 and HFA 134a or only HFA 134a are preferred.

If a mixture of the propellent gases HFA 227 and HFA 134a is used in the suspension formulations according to the invention, the weight ratios in which these two propellent gas components are used are freely variable.

If one or more other propellent gases, selected from the group consisting of propane, butane, pentane, dimethylether, CHC1F₂, CH₂F₂,CF₃CH₃, isobutane, isopentane and neopentane are used in addition to the propellent gases HFA 227 and/or HFA 134a in the suspension formulations according to the invention, the amount of this additional propellent gas component is preferably less than 50 %, preferably less than 40%, particularly preferably less than 30%.

The suspensions according to the invention preferably contain an amount of tiotropium bromide form such that the amount of tiotropium cation is between 0.001 and 0.8%, preferably between 0.08 and 0.5%, and particularly preferably between 0.2 and 0.4% according to the invention.

Unless stated to the contrary, the percentages given within the scope of the present invention are always percent by weight.

In some cases, the term suspension formulation is used within the scope of the present invention instead of the term suspension. The two terms are to be regarded as equivalent within the scope of the present invention.

The propellant-containing inhalable aerosols or suspension formulations according to the invention may also contain other constituents such as surface-active agents (surfactants), adjuvants, antioxidants or flavourings.

The surface-active agents (surfactants) optionally present in the suspensions according to the invention are preferably selected from the group consisting of Polysorbate 20, Polysorbate 80, Myvacet 9-45, Myvacet 9-08, isopropyl myristate, oleic acid, propyleneglycol, polyethyleneglycol, Brij, ethyl oleate, glyceryl trioleate, glyceryl monolaurate, glyceryl monooleate, glyceryl monostearate, glyceryl monoricinoleate, cetylalcohol, sterylalcohol, cetylpyridinium chloride, block polymers, natural oil, ethanol and isopropanol. Of the above- mentioned suspension adjuvants Polysorbate 20, Polysorbate 80, Myvacet 9-45, Myvacet 9-08 or isopropyl myristate are preferably used. Myvacet 9-45 or isopropyl myristate are most preferably used.

If the suspensions according to the invention contain surfactants these are preferably used in an amount of 0.0005 - 1 %, particularly preferably 0.005 - 0.5 %.

The adjuvants optionally contained in the suspensions according to the invention are preferably selected from the group consisting of alanine, albumin, ascorbic acid, aspartame, betaine, cysteine, phosphoric acid, nitric acid, hydrochloric acid, sulphuric acid and citric acid. Ascorbic acid, phosphoric acid, hydrochloric acid or citric acidare preferably used, while hydrochloric acid or citric acid is most preferably used.

If adjuvants are present in the suspensions according to the invention, these are preferably used in an amount of 0.0001-1.0 %, preferably 0.0005-0.1 %, particularly preferably 0.001-0.01 %, while an amount of 0.001-0.005 % is particularly important according to the invention.

The antioxidants optionally contained in the suspensions according to the invention are preferably selected from the group consisting of ascorbic acid, citric acid, sodium edetate, editic acid, tocopherols, butylhydroxytoluene, butylhydroxyanisol and ascorbylpalmitate, while tocopherols, butylhydroxytoluene, butylhydroxyanisol or ascorbylpalmitate are preferably used.

The flavourings optionally contained in the suspensions according to the invention are preferably selected from the group consisting of peppermint, saccharine, Dentomint, aspartame and ethereal oils (for example cinnamon, aniseed, menthol, camphor), of which peppermint or Dentomint® are particularly preferred.

With a view to administration by inhalation it is essential to provide the active substances in finely divided form. For this purpose, the crystalline tiotropium bromide forms according to the invention are obtained in finely divided form using me thods known in the prior art. Methods of micronising active substances are known in the art. Preferably after micronising the active substance has a mean particle size of 0.5 to 10µm, preferably 1 to 6µm, particularly preferably 1.5 to 5µm. Preferably at least 50%, preferably at least 60%, particularly preferably at least 70% of the particles of active substance have a particle size which is within the size ranges mentioned above. Particularly preferably at least 80%, most preferably at least 90% of the particles of active substance have a particle size which is within the size ranges mentioned above.

In another aspect the present invention relates to suspensions which contain only one of the two active substances according to the invention without any other additives.

The suspensions according to the invention may be prepared using methods known in the art. For this, the constituents of the formulation are mixed with the propellent gas or gases (optionally at low temperatures) and filled into suitable containers.

The above-mentioned propellant-containing suspensions according to the invention may be administered using inhalers known in the art (pMDIs = pressurized metered dose inhalers). Accordingly, in another aspect, the present invention relates to pharmaceutical compositions in the form of suspensions as hereinbefore described combined with one or more inhalers suitable for administering these suspensions. Moreover the present invention relates to inhalers, characterised in that they contain the propellant-containing suspensions according to the invention described hereinbefore.

The present invention also relates to containers (cartridges) which when fitted with a suitable valve can be used in a suitable inhaler and which contain one of the above-mentioned propellant-containing suspensions according to the invention. Suitable containers (cartridges) and processes for filling these cartridges with the propellant-containing suspensions according to the invention are known in the art.

In view of the pharmaceutical activity of tiotropium the present invention also relates to the use of the suspensions according to the invention for preparing a pharmaceutical composition for inhalation or nasal administration, preferably for preparing a pharmaceutical composition for inhalative or nasal treatment of diseases in which anticholinergics may develop a therapeutic benefit.

Particularly preferably the present invention also relates to the use of the suspensions according to the invention for preparing a pharmaceutical composition for the inhalative treatment of moderate persistent asthma as specified hereinbefore.

The Examples that follow serve to illustrate the present invention in more detail, by way of example, without restricting it to their contents.

### Examples of aerosol suspension formulations

Suspensions containing other ingredients in addition to active substance and propellent gas:

### Formulation Example 8:

| **constituents** | **concentration [% w/w]** |
|---|---|
| tiotropium bromide mono hydrate | 0.08 |
| oleic acid | 0.005 |
| HFA-227 | ad 100 |

### Formulation Example 9:

| **constituents** | **concentration [% w/w]** |
|---|---|
| tiotropium bromide monohydrate | 0.04 |
| oleic acid | 0.01 |
| HFA-227 | 60.00 |
| HFA-134a | ad 100 |

### Formulation Example 10:

| **constituents** | **concentration [% w/w]** |
|---|---|
| tiotropium bromide monohydrate | 0.04 |
| isopropylmyristate | 1.00 |
| HFA-227 | ad 100 |

### Formulation Example 11:

| **constituents** | **concentration [% w/w]** |
|---|---|
| tiotropium bromide monohydrate | 0.04 |
| Myvacet 9-45 | 0.3 |
| HFA-227 | ad 100 |

### Formulation Example 12:

| **constituents** | **concentration [% w/w]** |
|---|---|
| tiotropium bromide anhydrate | 0.04 |
| Myvacet 9-45 | 0.1 |
| HFA-227 | 60.00 |
| HFA-134a | ad 100 |

### Formulation Example 13:

| **constituents** | **concentration [% w/w]** |
|---|---|
| tiotropium bromide anhydrate | 0.04 |
| Polysorbate 80 | 0.04 |
| HFA-227 | ad 100 |

### Formulation Example 14:

| **constituents** | **concentration [% w/w]** |
|---|---|
| tiotropium bromide anhydrate | 0.02 |
| Polysorbate 20 | 0.20 |
| HFA-227 | ad 100 |

### Formulation Example 15:

| **constituents** | **concentration [% w/w]** |
|---|---|
| tiotropium bromide anhydrate | 0.04 |
| Myvacet 9-08 | 01.00 |
| HFA-227 | ad 100 |

### Formulation Example 16:

| **constituents** | **concentration [% w/w]** |
|---|---|
| tiotropium bromide anhydrate | 0.04 |
| isopropylmyristate | 0.30 |
| HFA-227 | 20.00 |
| HFA-134a | ad 100 |

### Formulation Example 17:

| **constituents** | **concentration [% w/w]** |
|---|---|
| tiotropium bromide anhydrate | 0.03 |
| HFA-227 | 60.00 |
| HFA-134a | ad 100 |

### Formulation Example 18:

| **constituents** | **concentration [% w/w]** |
|---|---|
| tiotropium bromide anhydrate | 0.04 |
| HFA-227 | ad 100 |

### Formulation Example 19:

| **constituents** | **concentration [% w/w]** |
|---|---|
| tiotropium bromide anhydrate | 0.04 |
| HFA-134a | ad 100 |

### Formulation Example 20:

| **constituents** | **concentration [% w/w]** |
|---|---|
| tiotropium bromide anhydrate | 0.04 |
| HFA-227 | ad 100 |

### Formulation Example 21:

| | |
|---|---|
| **constituents** I | **concentration [% w/w]** |
| tiotropium bromide anhydrate | 0.04 |
| HFA-134a | ad 100 |

### Formulation Example 22:

| **constituents-** | **concentration [% w/w]** |
|---|---|
| tiotropium bromide anhydrate | 0.02 |
| HFA-227 | 20.00 |
| HFA-134a | 79.98 |

### Propellant-free aerosol formulations

It is particularly preferred to use the tiotropium salts **1** according to the invention to prepare propellant-free inhalable solutions and suspensions. The solvent used may be an aqueous or alcoholic, preferably an ethanolic solution. The solvent may be water on its own or a mixture of water and ethanol. The relative proportion of ethanol compared with water is not limited but the maximum is up to 70 percent by volume, more particularly up to 60 percent by volume and most preferably up to 30 percent by volume. The remainder of the volume is made up of water. The solutions or suspensions containing **1** are adjusted to a pH of 2 to 7, preferably 2 to 5, using suitable acids. More preferably the pH of the formulation is between 2.8 and 3.05, preferably between 2.85 and 3.0, and most preferably 2.9.

The pH may be adjusted using acids selected from inorganic or organic acids. Examples of particularly suitable inorganic acids include hydrochloric acid, hydrobromic acid, nitric acid, sulphuric acid and/or phosphoric acid. Examples of particularly suitable organic acids include ascorbic acid, citric acid, malic acid, tartaric acid, maleic acid, succinic acid, fumaric acid, acetic acid, formic acid and/or propionic acid etc. Preferred inorganic acids are hydrochloric and sulphuric acids. It is also possible to use the acids which have already formed an acid addition salt with one of the active substances. Of the organic acids, ascorbic acid, fumaric acid and citric acid are preferred. If desired, mixtures of the above acids may be used, particularly in the case of acids which have other properties in addition to their acidifying qualities, e.g. as flavourings, antioxidants or complexing agents, such as citric acid or ascorbic acid, for example. According to the invention, it is particularly preferred to use hydrochloric acid to adjust the pH.

According to the invention, the addition of editic acid (EDTA) or one of the known salts thereof, sodium edetate, as stabiliser or complexing agent is unnecessary in the present formulation. Other embodiments may contain this compound or these compounds. In a preferred embodiment the content based on sodium edetate is less than 100mg/100ml, preferably less than 50mg/100 ml, more preferably less than 20mag/100 ml. Generally, inhalable solutions in which the content of sodium edetate is from 0 to 10mg/100ml are preferred.

Co-solvents and/or other excipients may be added to the propellant-free inhalable solutions which may be used according to the invention. Preferred co-solvents are those which contain hydroxyl groups or other polar groups, e.g. alcohols - particularly isopropyl alcohol, glycols - particularly propyleneglycol, polyethyleneglycol, polypropyleneglycol, glycolether, glycerol, polyoxyethylene alcohols and polyoxyethylene fatty acid esters. The terms excipients and additives in this context denote any pharmacologically acceptable substance which is not an active substance but which can be formulated with the active substance or substances in the pharmacologically suitable solvent in order to improve the qualitative properties of the active substance formulation. Preferably, these substances have no pharmacological effect or, in connection with the desired therapy, no appreciable or at least no undesirable pharmacological effect. The excipients and additives include, for example, surfactants such as soya lecithin, oleic acid, sorbitan esters, such as polysorbates, polyvinylpyrrolidone, other stabilisers, complexing agents; antioxidants and/or preservatives which guarantee or prolong the shelf life of the finished pharmaceutical formulation, flavourings, vitamins and/or other additives known in the art. The additives also include pharmacologically acceptable salts such as sodium chloride as isotonic agents.

The preferred excipients include antioxidants such as ascorbic acid, for example, provided that it has not already been used to adjust the pH, vitamin A, vitamin E, tocopherols and similar vitamins and provitamins occurring in the human body.

Preservatives may be used to protect the formulation from contamination with pathogens. Suitable preservatives are those which are known in the art, particularly cetyl pyridinium chloride, benzalkonium chloride or benzoic acid or benzoates such as sodium benzoate. Of particular imporatnce is benzalkonium chloride in concentrations of up to 50mg/100ml, more preferably between 5 and 20mg/100ml, even more preferably 8-15 mg/100ml of the formulation.

Preferred formulations contain, in addition to the solvent water and the tiotropium salts 1, only benzalkonium chloride and sodium edetate. In another preferred embodiment, no sodium edetate is present.

The propellant-free inhalable solutions which may be used within the scope of the invention are administered in particular using inhalers of the kind which are capable of nebulising a small amount of a liquid formulation in the therapeutic dose within a few seconds to produce an aerosol suitable for therapeutic inhalation. Within the scope of the present invention, preferred inhalers are those in which a quantity of less than 100µL, preferably less than 50µL, more preferably between 10 and 30µL of active substance, solution can be nebulised in preferably one spray action to form an aerosol with an average particle size of less than 20µm, preferably less than 10µm, in such a way that the inhalable part of the aerosol corresponds to the therapeutically effective quant ity.

An apparatus of this kind for propellant-free delivery of a metered quantity of a liquid pharmaceutical composition for inhalation is described for example in International Patent Application WO 91/14468 and also in WO 97/12687 (cf. in particular Figures 6a and 6b). The nebulisers (devices) described therein are also known by the name Respimat®.

The concentration of the tiotropium salt based on the proportion of tiotropium in the finished pharmaceutical preparation depends on the therapeutic effect sought. For most of the complaints which respond to tiotropium the concentration of tiotropium is between 0.01g per 100 ml of formulation and 0.06 g per 100 ml of formulation. An amount of 0.015 g /100 ml to 0.055 g /100 ml is preferred, an amount of from 0.02 g /100 ml to 0.05 g /100 ml is more preferred. Most preferred in the instant invention is an amount of from 0.023 ± 0.001g per 100 ml of formulation up to 0.045 ± 0.001g per 100 ml of formulation.

### Examples of propellant-free aerosol formulations

**100 ml of pharmaceutical preparation contain:**

| Example | tiotropium* | corresponds to tiotropium monohydrate | Amount of benzalkonium chloride | Amount of disodium edetate | pH, adjusted with HCl (1N) |
|---|---|---|---|---|---|
| 23 | 22.624 mg | 28.267 mg | 10 mg | 10 mg | 2.9 |
| 24 | 45.249 mg | 56.534 mg | 10 mg | 10 mg | 2.9 |
| 25 | 22.624 mg | 28.267 mg | 10 mg | 10 mg | 2.8 |
| 26 | 45.249 mg | 56.534 mg | 10 mg | 10 mg | 2.8 |
| 27 | 22.624 mg | 28.267 mg | 10 mg | 10 mg | 3.0 |
| 28 | 45.249 mg | 56.534 mg | 10 mg | 10 mg | 3.0 |
| 29 | 22.624 mg | 28.267 mg | 10 mg | 10 mg | 2.7 |
| 30 | 45.249 mg | 56.534 mg | 10 mg | 10 mg | 2.7 |
| 31 | 22.624 mg | 28.267 mg | 10 mg | 10 mg | 3.1 |
| 32 | 45.249 mg | 56.534 mg | 10 mg | 10 mg | 3.1 |

| | | | | | |
|---|---|---|---|---|---|
| *the amount specified refers to the tiotropium cation as the active entity of tiotropium bromide; 1 mg tiotropium corresponds to 1.2494 mg tiotropium bromide monohydrate | | | | | |

The remainder of the formulations 23-28 is purified water or water for injections at a density of 1.00 g/cm³ at a temperature of 15°C to 31°C.

If the formulations mentioned hereinbefore are delivered with the Respimat device 2 actuations of the device deliver 22.1µl of the formulation. Two actuations of the device, therefore, deliver with the formulations according to examples 23, 25, and 27 a dose of 5µg tiotropium (based on calculation for cation). Two actuations of the device deliver with the formulations according to examples 24, 26, and 28 a dose of 10µg tiotropium (based on calculation for cation).

Depending on the condition of the patient, also 3 or 4 actuations may for instance be administered.

### Further Examples 33 to 42:

Analogous to Examples 23 to 32, but with 8 mg of sodium edetate.

### Further Examples 43 to 52:

Analogous to Examples 23 to 32, but with 12 mg of sodium edetate.

### Further Examples 53 to 62:

Analogous to Examples 23 to 32, but with 8 mg of benzalkonium chloride.

### Further Examples 63 to 72:

Analogous to Examples 23 to 32, but with 12 mg of benzalkonium chloride.

Of the Examples 23 to 32, formulation 23 to 28 are of particular interest, with formulation examples 23-24 being of utmost importance.

## Claims

1. Use of tiotropium salts **1** 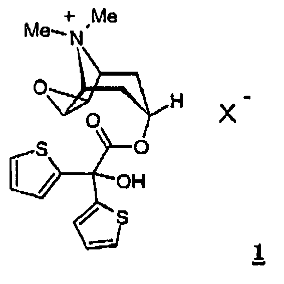 wherein
X⁻ denotes an anion with a single negative charge, preferably an anion which is selected from among chloride, bromide, iodide, sulphate, phosphate, methanesulphonate, nitrate, maleate, acetate, citrate, fumarate, tartrate, oxalate, succinate, benzoate and p-toluenesulphonate, optionally in form of the hydrates and/or solvates thereof,
for the manufacture of a medicament for the treatment of asthma in the severity GINA step 3.

2. Use according to claim 1, for the manufacture of a medicament for the treatment of moderate persistent asthma in patients showing daily symptoms despite of treatment with inhaled corticosteroids.

3. Use according to claim 1 or 2, for the manufacture of a medicament for the treatment of moderate persistent asthma in patients showing persistent symptoms despite of treatment with inhaled beta-2-agonists in patients bearing the Arg/Arg genotype at codon 16 of the ADRB2 gene (chromosome 5).

4. Use according to one of claims 1 or 2, for the manufacture of a medicament for the treatment of moderate persistent asthma in patients bearing the Arg/Arg genotype at codon 16 of the ADRB2 gene (chromosome 5) showing daily symptoms despite of combined treatment with inhaled corticosteroids and long-acting beta-2-agonists

5. Use according to one of claims 1 to 4, for the manufacture of a medicament for the treatment of moderate persistent asthma wherein the patients are children.

6. Use according to one of claims 1 to 5, for the manufacture of a medicament for the maintenance treatment of moderate persistent asthma and the prevention of broncho-obstructive symptoms in patients who are not adequately controlled by maintenance controller treatment with inhaled corticosteroids and long-acting beta-2-agonaists.

7. Use according to one of claims 1 to 6, for the manufacture of a medicament for the third-line maintenance controller therapy for the treatment of moderate persistent asthma.

8. Use according to one of claims 1 to 7, wherein per each individual dose preferably 1- 20 µg of tiotropium 1' are administered.

## Patentansprüche

1. Verwendung von Tiotropiumsalzen **1** 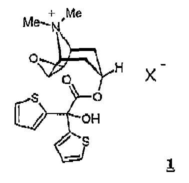 worin
X⁻ ein Anion mit einer einzelnen negativen Ladung bezeichnet, bevorzugt ein Anion, das ausgewählt ist aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, gegebenenfalls in Form der Hydrate und/oder Solvate hiervon,
zur Herstellung eines Arzneimittels zur Behandlung von Asthma im Schweregrad GINA Stufe3.

2. Verwendung nach Anspruch 1 zur Herstellung eines Arzneimittels zur Behandlung von moderatem persistierendem Asthma bei Patienten, die trotz der Behandlung mit inhalierten Kortikosteroiden täglich Symptome zeigen.

3. Verwendung nach Anspruch 1 oder 2 zur Herstellung eines Arzneimittels zur Behandlung von moderatem persistierendem Asthma bei Patienten, die persistente Symptome zeigen trotz der Behandlung mit inhalierten beta-2-Agonisten bei Patienten, die den Arg/Arg-Genotyp auf Codon 16 des ADRB2-Gens (Chromosom 5) tragen.

4. Verwendung nach einem der Ansprüche 1 oder 2 zur Herstellung eines Arzneimittels zur Behandlung von moderatem persistierendem Asthma bei Patienten, die den Arg/Arg-Genotyp auf Codon 16 des ADRB2-Gens (Chromosom 5) tragen, die trotz der kombinierten Behandlung mit inhalierten Kortikosteroiden und langwirkenden beta-2-Agonisten täglich Symptome zeigen.

5. Verwendung nach einem der Ansprüche 1 bis 4 zur Herstellung eines Arzneimittels zur Behandlung von moderatem persistierendem Asthma, wobei die Patienten Kinder sind.

6. Verwendung nach einem der Ansprüche 1 bis 5 zur Herstellung eines Arzneimittels zur zustandserhaltenden ("maintenance") Behandlung von moderatem persistierendem Asthma und der Vorbeugung von bronchoobstruktiven Symptomen bei Patienten, die nicht angemessen unter Kontrolle stehen durch zustandserhaltende Kontroll-Behandlung mit inhalierten Kortikosteroiden und langwirkenden beta-2-Agonisten.

7. Verwendung nach einem der Ansprüche 1 bis 6 zur Herstellung eines Arzneimittels zur zustandserhaltenden ("maintenance") Third-Line-Kontrolltherpie zur Behandlung von moderatem persistierendem Asthma.

8. Verwendung nach einem der Ansprüche 1 bis 7, wobei mit jeder einzelnen Dosis bevorzugt 1 bis 20 µg Tiotropium 1' verabreicht werden.

## Revendications

1. Utilisation de sels de tiotropium 1 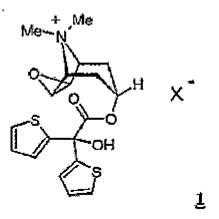 dans laquelle X⁻ désigne un anion doté d'une charge négative simple, de préférence un anion qui est choisi parmi le chlore, le brome, l'iode, le sulfate, le phosphate, le méthane-sulfonate, le nitrate, le maléate, l'acétate, le citrate, le fumarate, le tartrate, l'oxalate, le succinate, le benzoate et le p-toluène-sulfonate, éventuellement sous la forme de leurs hydrates et/ou solvates,
pour la production d'un médicament destiné au traitement de l'asthme de sévérité GINA, étape 3.

2. Utilisation selon la revendication 1, pour la production d'un médicament destiné au traitement de l'asthme modéré persistant chez des patients présentant des symptômes quotidiens malgré un traitement par corticostéroïdes inhalés.

3. Utilisation selon la revendication 1 ou 2, pour le production d'un médicament destiné au traitement de l'asthme modéré persistant chez des patients présentant des symptômes persistants malgré un traitement avec des bêta-2-agonistes inhalés chez des patients portant le génotype Arg/Arg au codon 16 du gène ADRB2 (chromosome 5).

4. Utilisation selon l'une des revendications 1 ou 2, pour la production d'un médicament destiné au traitement de l'asthme modéré persistant chez des patients portant le génotype Arg/Arg au codon 16 du gène ADRB2 (chromosome 5) présentant des symptômes quotidiens malgré un traitement combiné avec des corticostéroïdes inhalés et des bêta-2-agonistes à longue durée d'action.

5. Utilisation selon la revendication 1 à 4, pour la production d'un médicament destiné au traitement de l'asthme modéré persistant dans laquelle les patients sont des enfants.

6. Utilisation selon l'une des revendications 1 à 5, pour la production d'un médicament destiné au traitement d'entretien de l'asthme modéré persistant et pour la prévention des symptômes broncho-obstructifs chez des patients dont l'état n'est pas contrôlé de façon adéquate par un traitement d'entretien de contrôle avec des corticostéroïdes inhalés et des bêta-2-agonistes à longue durée d'action.

7. Utilisation selon l'une des revendications 1 à 6, pour la production d'un médicament pour le traitement d'entretien de contrôle de troisième ligne pour le traitement de l'asthme modéré persistant.

8. Utilisation selon l'une des revendications 1 à 7, dans laquelle pour chaque dose individuelle, de préférence 1 à 20 µg de tiotropium 1' sont administrés.
